(19) Europäisches Patentamt
European Patent Office
Office européen des brevets

(11) EP 3 377 119 B1

(12) # FASCICULE DE BREVET EUROPEEN

(45) Date de publication et mention de la délivrance du brevet:
**18.11.2020 Bulletin 2020/47**

(21) Numéro de dépôt: **16809994.3**

(22) Date de dépôt: **18.11.2016**

(51) Int Cl.:
***A61L 2/18*** (2006.01) ***A61L 2/20*** (2006.01)
***A61L 2/22*** (2006.01) ***C01B 15/037*** (2006.01)

(86) Numéro de dépôt international:
**PCT/FR2016/053016**

(87) Numéro de publication internationale:
**WO 2017/085428 (26.05.2017 Gazette 2017/21)**

(54) **SOLUTION AQUEUSE DE PEROXYDE D'HYDROGENE COMPRENANT UN STABILISANT SPECIFIQUE**

WÄSSRIGEN WASSERSTOFFPEROXIDLÖSUNG MIT SPEZIFISCHEM STABILISATOR

HYDROGEN PEROXIDE AQUEOUS SOLUTION CONTAINING A SPECIFIC STABILIZER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité: **18.11.2015 FR 1561086**

(43) Date de publication de la demande:
**26.09.2018 Bulletin 2018/39**

(73) Titulaire: **Arkema France**
**92700 Colombes (FR)**

(72) Inventeurs:
- **ZYDOWICZ, Philippe**
**69800 Saint Priest (FR)**
- **LARNICOL, Pierre**
**91370 Verriere le Buisson (FR)**

(74) Mandataire: **Arkema Patent**
**Arkema France**
**DRD-DPI**
**420, rue d'Estienne d'Orves**
**92705 Colombes Cedex (FR)**

(56) Documents cités:
**EP-A1- 1 762 252** **EP-A1- 2 338 961**
**WO-A1-2013/026971** **WO-A1-2016/082897**
**WO-A1-2016/082897** **WO-A2-2010/004161**
**CN-A- 102 256 713** **US-A- 5 759 440**
**US-A1- 2011 052 445**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 377 119 B1

## Description

### DOMAINE DE L'INVENTION

La présente invention se rapporte à une solution aqueuse de peroxyde d'hydrogène comportant un stabilisant spécifique lui permettant d'obtenir des propriétés significativement améliorées par rapport aux produits de l'état de la technique.

L'invention se rapporte aussi à un procédé de désinfection, voire de stérilisation, mettant en œuvre la solution aqueuse selon la présente invention. L'invention a enfin pour objet l'utilisation d'une telle solution aqueuse de désinfection spécifiquement stabilisée pour désinfecter un emballage.

### ARRIERE-PLAN TECHNIQUE

Pour désinfecter ou stériliser un emballage, une technique de désinfection ou stérilisation consiste à asperger ledit emballage à l'aide d'un spray d'une solution aqueuse de peroxyde d'hydrogène. Avec cette solution, une certaine quantité de stabilisant(s) est nécessaire, ce qui conduit à la présence, dans la solution de peroxyde d'hydrogène, de résidus provenant de la décomposition ou de la présence des stabilisants (outre ceux provenant du peroxyde d'hydrogène).

Les stabilisants sont essentiels à la solution aqueuse de peroxyde d'hydrogène car ils empêchent la dégradation du peroxyde d'hydrogène (réaction exothermique de dismutation en eau et en oxygène). Ce besoin en stabilisant est d'autant plus important que la teneur en peroxyde d'hydrogène augmente. Ainsi, pour que des solutions de peroxyde d'hydrogène soient efficaces dans la désinfection ou la stérilisation, la teneur en peroxyde d'hydrogène doit être élevée, et par conséquent la teneur en stabilisant également.

Or, les résidus résultant de ces stabilisants peuvent boucher la buse ou les buses et les conduits de l'appareil de spray (pulvérisation).

Bien entendu, il est possible de nettoyer l'appareil de pulvérisation mais cela entraîne l'arrêt du dispositif de désinfection/stérilisation ainsi que des coûts additionnels non négligeables, outre le fait que le pulvérisateur peut être irrémédiablement endommagé. Seules des solutions présentant de très faibles teneurs en résidus permettraient donc l'utilisation de telles solutions aqueuses de peroxyde d'hydrogène par pulvérisation.

On connaît à l'heure actuelle des documents qui proposent de nouveaux types de stabilisants, tels que dans le document EP 1926502 ou le document WO 2015078830, ou dans les documents WO2010/004161, EP 10177812, WO 2010/073976, US 5759440; mais aucune de ces solutions ne permet d'atteindre un niveau extrêmement faible de résidus indispensable à l'utilisation par pulvérisation.

Ainsi, il existe un besoin de disposer d'une solution de désinfection/stérilisation efficace, stable dans le temps (conservation du titre en peroxyde d'hydrogène) présentant une très faible teneur en résidus.

### RESUME DE L'INVENTION

La présente invention a pour objectif de remédier aux inconvénients précités. Elle propose à cette fin l'utilisation de stabilisants d'une famille chimique bien particulière qui permettent, tout en remplissant idéalement leur fonction première (stabilisation du peroxyde d'hydrogène) de garantir un niveau particulièrement faible de résidus de sorte que les opérations de désinfection/stérilisation d'un emballage n'entraînent aucun risque d'endommagement, même à très long terme, des appareils nécessaires à ces opérations (sans nécessiter par ailleurs d'opérations de lavage/nettoyage).

De façon particulièrement surprenante, la composition de désinfection/stérilisation selon l'invention assure la stabilité du peroxyde d'hydrogène (principe actif de la composition) à température ambiante même en présence d'une faible contamination métallique. Les résultats de stabilité de la composition selon l'invention montrent une amélioration très significative des propriétés de stabilité, tout en offrant une très faible quantité de résidus susceptibles de détériorer les dispositifs utilisés pour dispenser (projection, pulvérisation, nébulisation, vaporisation, etc...) la composition de peroxyde d'hydrogène. De manière surprenante, une telle stabilité est observée même pour des teneurs en peroxyde d'hydrogène importantes, comme celles exigées pour assurer une désinfection/stérilisation efficace.

En outre, la composition de désinfection/stérilisation selon l'invention permet la désinfection d'un emballage destiné à emballer des aliments pour humains ou animaux, des produits cosmétiques, des produits pharmaceutiques ou vétérinaires.

Ainsi la présente invention concerne une solution aqueuse de peroxyde d'hydrogène, comprenant de 1% à 98%, en particulier de 3% à 75%, de préférence de 20 à 98%, de préférence de 20 à 75%, de préférence de 20% à 65%, de préférence de 20 à 50% encore préférentiellement de 22% à 38% en poids de la solution, de peroxyde d'hydrogène et de 0,01 à 7 mg/kg (milligramme par kilogramme) de préférence de 0,5 à 7 mg/kg, encore préférentiellement de 1,5 à 5 mg/kg, en poids de la solution, d'un stabilisant, caractérisée en ce que le stabilisant est choisi dans la famille

des acides aminopolycarboxyliques (APCA) ou leurs sels, de formule générique :

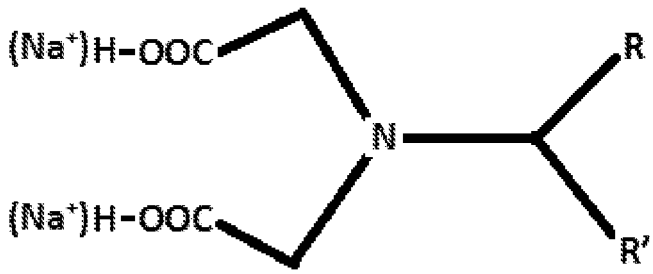

où R et R' sont choisis parmi les groupes fonctionnels suivants :

- alkyls en C1 à C3, de préférence un groupement méthyl,
- acides carboxyliques en C1 à C3.

Il est bien entendu que la quantité de stabilisants indiquées ci-dessus se rapporte à une quantité de matière active du ou des stabilisants, ceux-ci pouvant être disponibles, éventuellement dans le commerce, sous forme de solution diluée dans de l'eau.

La présente invention trouve à s'appliquer en particulier pour toute mise en œuvre de la solution aqueuse selon l'invention sous forme vaporisée, tels que notamment dans l'application dénommée « aseptique spray » connue de l'homme du métier et plus généralement à la désinfection/stérilisation via vaporisation de la solution de peroxyde d'hydrogène.

Dans la suite, sauf mention contraire, les pourcentages sont donnés en valeurs massiques.

Il est bien entendu que le terme « stabilisant » se rapporte dans la présente demande à tout composant ayant pour fonction d'assurer la stabilité dans le temps du peroxyde d'hydrogène présent dans la solution aqueuse de sorte que ce dernier ne se décompose pas ou peu et que le titre de la solution reste sensiblement à l'identique au fil du temps, lors par exemple d'un stockage. A titre d'exemple non limitatif de tels stabilisants utilisés dans l'état de la technique, on pourra citer les acides phosphoniques et leurs sels, tels que l'acide amino-tris methylene phosphonique, l'acide hydroxy ethylene diphosphonique, ou l'acide diethylene triamine penta methylphosphonique, les polyphosphates, les pyrophosphates tels que le pyrophosphate acide de sodium, l'acide phosphorique et ses sels, le stannate de sodium, les acides carboxyliques, les dérivés de l'acide éthylenediamine tetracétique.

La « désinfection » d'un emballage au sens de l'invention peut s'entendre d'une destruction partielle des microorganismes présents sur la surface de l'emballage afin de ralentir leur prolifération, à une quasi-totale extermination desdits microorganismes, selon l'application visée. Dans ce dernier cas, on parle généralement de « stérilisation ». La composition aqueuse de peroxyde d'hydrogène selon l'invention convient à ces deux utilisations.

Afin d'assurer une désinfection/stérilisation adéquate, une teneur minimale de 20%, de préférence 22% de peroxyde d'hydrogène par rapport au poids totale de la composition est requise.

De préférence la solution selon la présente invention comprend de 20 à 98%, de préférence de 20 à 75%, de préférence de 20% à 65%, de préférence de 20 à 50%, encore préférentiellement de 22% à 38% de peroxyde d'hydrogène par rapport au poids totale de la composition.

Une teneur en peroxyde d'hydrogène de 22% à 38% par rapport au poids totale de la composition est particulièrement appropriée pour la désinfection/stérilisation des emballages.

On entend par « résidu(s) », « résidu(s) sec(s) » ou « résidus secs d'évaporation» de la solution selon la présente invention, la matière solide recueillie après évaporation de la composition de désinfection à 110°C par la méthode gravimétrique qui sera décrite plus loin.

De préférence, la teneur en résidu de la solution selon la présente invention est inférieur à 7 mg/kg (milligramme par kilogramme de solution de peroxyde d'hydrogène), de préférence inférieure à 6 mg/kg, de préférence inférieure à 5 mg/kg, de préférence inférieure à 4 mg/kg, de préférence inférieure à 3 mg/kg et encore préférentiellement inférieure à 2 mg/kg. Ces résidus peuvent notamment provenir du ou des stabilisants du peroxyde d'hydrogène et/ou de composés mis en œuvre lors de la fabrication du peroxyde d'hydrogène utilisé dans la présente invention, par exemple un agent anticorrosion, un acide minéral ou bien un additif connu de l'homme de l'art.

Néanmoins, la teneur en stabilisant(s) présent(s) initialement dans la solution aqueuse de peroxyde d'hydrogène est prépondérante pour déterminer la teneur de résidus à sec présents *in fine.* Ainsi, l'invention a pour objectif de réduire, voire d'éliminer, la présence de résidus provenant du ou des stabilisants du peroxyde d'hydrogène utilisés dans la solution aqueuse.

Pour ce faire, il est nécessaire que la teneur en stabilisant soit inférieure à 7 mg/kg, de préférence inférieure

à 6 mg/kg, de préférence inférieure à 5 mg/kg, de préférence inférieure à 4 mg/kg, de préférence inférieure à 3 mg/kg, de préférence inférieure à 2,5 mg/kg et encore préférentiellement de préférence inférieure à 2 mg/kg.

Afin de stabiliser le peroxyde d'hydrogène dans la solution, la teneur en stabilisant est de préférence supérieure à 0,5 mg/kg, de préférence supérieure à 1 mg/kg, et encore préférentiellement supérieure à 1,5 mg/kg. Avantageusement, le stabilisant représente de 1 à 2,5 mg/kg en poids de la solution aqueuse.

Ainsi, grâce à l'invention, il est possible d'obtenir une quantité de résidus secs inférieure à 2 mg/kg mais il est entendu que pour obtenir un tel résultat, le choix de la solution de peroxyde d'hydrogène est essentiel, autrement dit cette dernière doit comporter un niveau très faible de résidus à sec issus de la fabrication dudit peroxyde, évidemment inférieur à 2 mg/kg.

Selon un mode préféré de réalisation de l'invention, R ou R' consiste en un groupement méthyle et l'autre R ou R' consiste en un groupement acide méthanoïque (acide formique) ou son sel. Dans ce cas, le stabilisant est l'acide méthylglycinediacétique ou son sel.

Selon un autre mode préféré de réalisation de l'invention, R ou R' consiste en un groupement acide éthanoïque (acide acétique) ou son sel, et l'autre R ou R' consiste en un groupement d'acide propanoïque ou son sel. Dans ce cas, le stabilisant est l'acide glutamate diacétique ou son sel.

D'autres caractéristiques ou modes de réalisation de l'invention sont présentées ci-après :

- avantageusement, la solution aqueuse selon l'invention présente une conductivité de 10 à 100 μS/cm (micro Siemens par centimètre), préférentiellement de 15 à 50 μS/cm, et de manière encore plus préférée de 20 à 40 μS/cm.
- avantageusement, la solution aqueuse selon l'invention présente une acidité maximum de 1 mmol/kg (millimole par kilogramme).
- avantageusement, la perte de titre de ladite solution/stabilité du peroxyde d'hydrogène dans la solution, mesurée selon la méthode CEFIC-$H_2O_2$-AM-7161, est inférieure ou égale à 5%, de préférence inférieure ou égale à 3 %, et de manière préférée inférieure ou égale à 2% par rapport à la concentration initiale en peroxyde d'hydrogène.

Selon une possibilité offerte par l'invention, la solution aqueuse comprend, et de préférence consiste en :

- de 20 % à 50 % de peroxyde d'hydrogène,
- De 0,3 à 3 mg/kg, de préférence de 0,5 à 7 mg/kg en poids de la solution, d'un stabilisant tel que défini ci-dessus,
- optionnellement, de $1.10^{-4}$ % à $1.10^{-2}$ %, en poids de la solution, d'additif(s),
- du complément à 100 % d'eau.

Avantageusement, on peut préparer une composition de désinfection selon l'invention étant uniquement constituée d'eau, de peroxyde d'hydrogène et du stabilisant selon l'invention. De manière surprenante, cette simple composition de désinfection se révèle efficace pour empêcher la décomposition du peroxyde d'hydrogène lorsque la composition de désinfection est contaminée par des impuretés métalliques.

Comme la composition de désinfection selon l'invention présente intrinsèquement une très faible teneur en résidus secs d'évaporation (issus du stabilisant de peroxyde d'hydrogène), elle peut supporter une contamination par des impuretés sans que la teneur totale en résidus secs ne franchisse le seuil maximal acceptable imposé par les réglementations en vigueur en matière de conditionnement alimentaire (à l'heure actuelle fixé par le « Food Chemical Codex »).

La présente invention se rapporte également à l'utilisation de la solution aqueuse telle que définie ci-dessus pour la désinfection/stérilisation d'un produit.

De préférence, ledit produit à désinfecter est un choisi dans le groupe constitué par les emballages, les locaux, en particulier des locaux hospitaliers, et des produits comestibles, en particulier des fruits ou des légumes.

De préférence, ledit emballage est choisi dans le groupe constitué par un emballage alimentaire, tel qu'un emballage alimentaire pour humain ou animal, un emballage d'un produit cosmétique, un emballage d'un produit pharmaceutique et un emballage d'un produit vétérinaire.

De préférence, la désinfection/stérilisation dudit produit est réalisée par pulvérisation ou vaporisation de ladite solution sur ledit produit.

La présente invention se rapporte également à l'utilisation de la solution aqueuse telle que présentée ci-dessus pour la désinfection/stérilisation d'un emballage par pulvérisation de la solution de peroxyde d'hydrogène décrite ci-dessus ou pour la désinfection par vapeur de ladite solution de peroxyde d'hydrogène.

Dans la suite, le stabilisant choisi dans la famille des acides aminopolycarboxyliques (APCA) ou leurs sels, de formule générique :

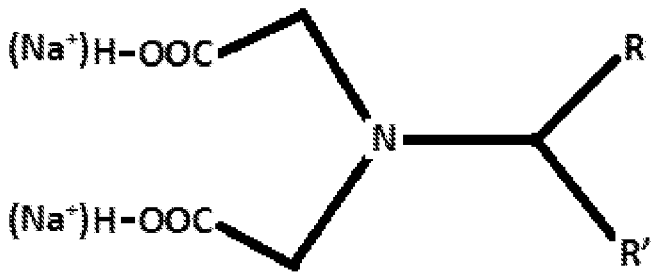

où R et R’ sont choisis parmi les groupes fonctionnels suivants :

- alkyls en C1 à C3, de préférence un groupement méthyl,
- acides carboxyliques en C1 à C3,

sera désigné éventuellement par l’expression le « stabilisant selon l’invention » englobant les acides susvisés et leurs sels.

L’invention se rapporte également à un procédé de désinfection/stérilisation d’un produit tel que défini ci-dessus, en particulier d’un emballage tel que défini ci-dessus, caractérisé en ce que ledit produit est mis en contact avec la solution aqueuse telle que décrite ci-dessus.

Ladite mise en contact permet de désinfecter/stériliser ledit produit, de préférence ledit emballage.

De préférence, ladite mise en contact est réalisée par pulvérisation ou vaporisation de ladite solution sur ledit produit, de manière particulièrement préférée par pulvérisation.

Avantageusement, la solution aqueuse subit, préalablement à sa mise en contact avec le stabilisant, une opération destinée à purifier ladite solution, telle que par exemple la distillation, un ou plusieurs passages au travers de résines échangeuses d’ion, la filtration ou par des techniques membranaires (microfiltration, ultrafiltration, nanofiltration) et/ou l’osmose inverse, cette dernière étant préférée parmi les techniques membranaires.

Dans le cadre de la présente invention, le passage de la solution aqueuse de peroxyde d’hydrogène par une opération préalable de purification peut consister en une combinaison d’opérations, c’est-à-dire incluant une ou plusieurs techniques de purification, notamment celles susvisées, connues de l’homme du métier.

Les modes de réalisation qui vont à présent être décrits se rapportent notamment tant à l’utilisation de la composition de désinfection/stérilisation qu’au procédé de désinfection selon l’invention. Ces différents modes peuvent avantageusement être combinés entre eux.

## DESCRIPTION DE MODES DE REALISATION DE L’INVENTION

L’invention est maintenant décrite plus en détail et de façon non limitative dans la description qui suit.

Pour fabriquer une solution aqueuse de peroxyde d’hydrogène selon l’invention, on peut mettre en présence le peroxyde d’hydrogène et le stabilisant selon l’invention par simple mélange. On peut en particulier solubiliser le stabilisant à l’état solide dans une solution aqueuse de peroxyde d’hydrogène. La fabrication de peroxyde d’hydrogène est une technique bien connue de l’homme du métier et ne constitue pas l’objet de la présente invention.

La présente invention se rapporte également à un procédé de fabrication d’une solution aqueuse de peroxyde d’hydrogène selon la présente invention comprenant une étape a) de mélange de :

- 20 à 98%, de préférence de 20 à 75%, de préférence de 20% à 65%, de préférence de 20 à 50% encore préférentiellement de 22% à 38%, en poids de la solution, de peroxyde d’hydrogène, et
- 0,5 à 7 mg/kg en poids de la solution, d’un stabilisant tel que défini ci-dessus.

De préférence, l’étape de mélange permet une solubilisation dudit stabilisant dans la solution aqueuse de peroxyde d’hydrogène.

De préférence, ledit procédé comprend en outre une étape a’), préalable à l’étape a), de purification de ladite solution aqueuse comprenant le peroxyde d’hydrogène.

## PARAMETRES MESURES ET METHODES UTILISEES

Lorsqu’il est fait référence à une méthode standard, les conditions de mise en œuvre sont celles décrites dans cette méthode, sauf adaptation explicitement mentionnée.

Le titre en peroxyde d’hydrogène dans la composition de désinfection est mesuré selon la méthode CEFIC-

$H_2O_2$-AM-7157 connue de l'homme du métier. Cette méthode consiste à titrer le peroxyde d'hydrogène dans une solution aqueuse d'acide sulfurique à l'aide d'une solution volumétrique standard de permanganate de potassium.

La stabilité du peroxyde d'hydrogène est évaluée par la méthode CEFIC-$H_2O_2$-AM-7161. Cette méthode consiste à déterminer le pourcentage de perte en peroxyde d'hydrogène, par mesure de la teneur en peroxyde d'hydrogène avant et après son chauffage à 96°C pendant 16 heures.

La teneur en résidus secs d'évaporation de la composition de désinfection est mesurée à 110°C par la méthode gravimétrique suivante :

1 - On place une capsule en platine de capacité voisine de 300-350 mL (millilitre) dans un four à moufle à 900°C pendant une heure. On refroidit la capsule dans un dessiccateur et on la pèse sur une balance de précision à 0,0001 g près. Cette masse est notée Y (en g).

2 - On pèse à 0,0001 g (gramme) près un bécher d'environ 500 mL et on note sa masse. On ajoute à ce bécher environ 300 g. On pèse à nouveau le bêcher, à 0,0001 g près. On note W1 (en gramme) la différence entre la masse du bécher avant et après l'addition de la prise d'échantillon.

3 - On transfère progressivement l'échantillon dans la capsule d'évaporation en platine (immergée dans de l'eau froide) au moyen d'une pompe péristaltique permettant d'obtenir un débit d'alimentation d'environ 75 grammes par heure. Une fois la prise d'essai transférée et le peroxyde d'hydrogène complètement décomposé, on évapore à sec l'échantillon dans un bain-marie. On renouvelle les opérations 2 et 3 deux fois afin d'obtenir un cumul de prise d'échantillons compris entre 750 et 1000 g. Cela permet d'obtenir une meilleure précision sur la détermination du résidu sec.

4 - On place ensuite la capsule platine dans un four à 110°C pendant au moins 1 heure.

5 - On refroidit la capsule dans un dessiccateur pendant 30 minutes puis on la pèse à 0,0001 g près. On note ce poids Z (en g).

6 - On calcule la teneur en résidu sec à l'aide de la formule :

$$\text{Résidu sec à 110°C (en g/kg)} = 1000 \times (Z-Y) / (W1 + W2 + W3)$$

dans laquelle

Z (en g) représente la masse de la capsule contenant le résidu sec après évaporation,
Y (en g) représente la masse de la capsule vide,
W1 , W2 et W3 (en g) représentent les masses des prises d'échantillon successives.

EXEMPLES

Les exemples suivants illustrent l'invention sans la limiter. En particulier, ils reproduisent le comportement de solutions aqueuses de peroxyde d'hydrogène dans des conditions de mise en œuvre, à savoir celles d'une machine de conditionnement aseptique pour stériliser un emballage.

Dans tous les exemples présentés ci-après, on considère que la quantité de peroxyde d'hydrogène dans la solution aqueuse est de 35%. Il faut noter que la stabilité est testée ici pour la solution aqueuse pure et en présence d'une contamination métallique en faible concentration (1 mg/litre de Fer). Pour ce dernier test, il sera admis que la valeur seuil maximale est fixée à 20% pour être conforme à l'invention.

Les résultats de quantités de résidus à sec incluent à la fois ceux provenant du peroxyde (c'est-à-dire issus essentiellement de sa fabrication) tout comme ceux provenant du ou des stabilisants, ces derniers étant les seuls que la présente invention entend éliminer, ou réduire très significativement, de la solution aqueuse (finale) de peroxyde d'hydrogène.

On utilise les produits de départ suivants :

Exemple 1

La solution aqueuse de l'exemple 1 comprend 0,1 mg/kg d'acide méthylglycinediacétique (ou de son sel).

Exemple 2

La solution aqueuse de l'exemple 2 comprend 1,1 mg/kg d'acide méthylglycinediacétique (ou de son sel).

Exemple 3

La solution aqueuse de l'exemple 3 comprend 3.3 mg/kg d'acide méthylglycinediacétique (ou de son sel).

Exemple 4

La solution aqueuse de l'exemple 4 comprend 1,1 mg/kg d'acide glutamate diacétique (ou de son sel).

Exemple 5 (comparatif)

La solution aqueuse de l'exemple 5, est un grade de peroxyde d'hydrogène actuellement commercialement (Valsterane® 35S) disponible pour les applications de désinfection des emballages alimentaires par pulvérisation, comprenant 4mg/kg d'un acide phosphonique en tant que stabilisant.

Exemple 6 (comparatif)

La solution aqueuse de l'exemple 6 est un autre grade de peroxyde d'hydrogène actuellement commercialement (Valsterane® 35SB) disponible pour les applications de désinfection des emballages alimentaires par pulvérisation, comprenant 16mg/kg d'un acide phosphonique en tant que stabilisant.

Exemple 7 (comparatif)

La solution aqueuse de l'exemple 7 comprend 1,1 mg/kg d'acide cyclohexanediaminotetraacétique (ou de son sel).

Exemple 8 (comparatif)

La solution aqueuse de l'exemple 8 comprend 1,1 mg/kg d'acide diplicolinique (ou de son sel).

Exemple 9 (comparatif)

La solution aqueuse de l'exemple 9 comprend 1,1 mg/kg d'acide diethylenetriaminepentaacétique (ou de son sel).

Exemple 10 (conforme à l'invention, domaine le plus préféré)

La solution aqueuse de l'exemple 10 comprend 2,3 mg/kg d'acide méthylglycinediacétique (ou de son sel).

Exemple 11 (conforme à l'invention, domaine préféré)

La solution aqueuse de l'exemple 11 comprend 4 mg/kg d'acide méthylglycinediacétique (ou de son sel).

Exemple 12 (conforme à l'invention)

La solution aqueuse de l'exemple 12 comprend 6 mg/kg d'acide méthylglycinediacétique (ou de son sel).
Les résultats obtenus sont indiqués dans le tableau ci-dessous :

| Ex | Stabilité* (16h/96°C) | Résidu sec à 110°C (mg/kg) | Stabilité* en condition de contamination (1 mg/L Fe) |
|---|---|---|---|
| 1 | 2.7 | 4 | 20 |
| 2 | 1.1 | 4.3 | 10 |
| 3 | 0.8 | 6.5 | 8 |
| 4 | 1.4 | 4.3 | 13 |
| 5 | 1 | 9 | 25 |
| 6 | 0.8 | 18 | 10 |

(suite)

| Ex | Stabilité* (16h/96°C) | Résidu sec à 110°C (mg/kg) | Stabilité* en condition de contamination (1 mg/L Fe) |
|---|---|---|---|
| 7 | 2 | 4.3 | 30 |
| 8 | 2.5 | 4.3 | 35 |
| 9 | 2 | 4.3 | 30 |
| 10 | 1 | 6 | 10 |
| 11 | 0.7 | 6.8 | 7 |
| 12 | 0.6 | 7 | 6 |
| *exprimée en % relatif de perte de titre $H_2O_2$ | | | |

On constate ainsi que seules les compositions selon l'invention, en particulier les compositions 2 à 4, présentent une excellente stabilité dans le temps (avec ou sans contamination) et une quantité de résidus à sec extrêmement faible.

## Revendications

**1.** Solution aqueuse de peroxyde d'hydrogène, comprenant :

- de 20 à 98% en poids de la solution, de peroxyde d'hydrogène,
et
- de 0,5 à 7 mg/kg, encore préférentiellement de 1,5 à 5 mg/kg
en poids de la solution, d'un stabilisant,
**caractérisée en ce que** le stabilisant est choisi dans la famille des acides aminopolycarboxyliques (APCA) ou leurs sels, de formule générique :

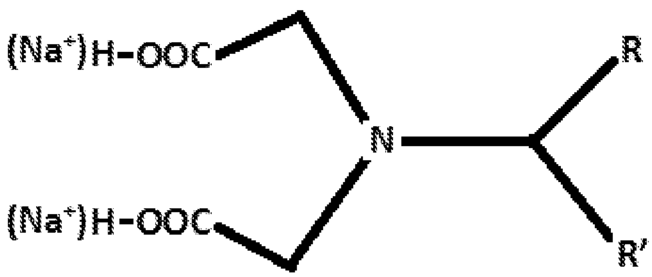

où R et R' sont choisis parmi les groupes fonctionnels suivants :
- alkyls en C1 à C3, de préférence un groupement méthyl,
- acides carboxyliques en C1 à C3.

**2.** Solution aqueuse selon la revendication 1, **caractérisée en ce qu'**elle comprend de 20 à 75%, de préférence de 20% à 65%, de préférence de 20 à 50%, encore préférentiellement de 22% à 38% en poids de la solution, de peroxyde d'hydrogène.

**3.** Solution aqueuse selon la revendication 1 ou 2, **caractérisée en ce que** R ou R' consiste en un groupement méthyle et l'autre R ou R' consiste en un groupement acide méthanoïque ou son el, de sorte le stabilisant consiste en l'acide méthylglycinediacétique ou son sel.

**4.** Solution aqueuse selon la revendication 1 ou 2, **caractérisée en ce que** R ou R' consiste en un groupement acide éthanoïque ou son sel, l'autre R ou R' consiste en un groupement d'acide propanoïque ou son sel, de sorte que le stabilisant consiste en l'acide glutamate diacétique ou son sel.

**5.** Solution aqueuse selon l'une quelconque des revendications précédentes, **caractérisée en ce qu'**elle présente conductivité de 10 à 100 $\mu$S/cm, préférentiellement de 15 à 50 $\mu$S/cm, et de manière encore plus préférée de 20

à 40 $\mu$S/cm.

**6.** Solution aqueuse selon l'une quelconque des revendications précédentes, **caractérisée en ce qu'**elle présente une acidité maximum de 1 mmol/kg.

**7.** Solution aqueuse selon l'une quelconque des revendications précédentes, **caractérisée en ce qu'**elle présente une teneur en résidu sec à 110°C d'au plus 7 mg/kg, de préférence inférieure à 6 mg/kg, de préférence inférieure à 5 mg/kg, de préférence inférieure à 4 mg/kg, de préférence inférieure à 3 mg/kg et encore préférentiellement inférieure à 2 mg/kg.

**8.** Solution aqueuse selon l'une quelconque des revendications précédentes, **caractérisée en ce que** la perte de titre de ladite solution, mesurée selon la méthode CEFIC-$H_2O_2$-AM-7161, est inférieure ou égale à 5%, de préférence inférieure ou égale à 3 %, et de manière préférée inférieure ou égale à 2% par rapport à la concentration initiale en peroxyde d'hydrogène.

**9.** Solution aqueuse selon l'une quelconque des revendications précédentes, **caractérisée en ce que** le stabilisant représente de 0,1 à 5 mg/kg, en poids de la solution, de préférence de 1 à 2,5 mg/kg, en poids de la solution.

**10.** Solution aqueuse selon l'une quelconque des revendications précédentes, comprenant :

- de 20 % à 50 % de peroxyde d'hydrogène, en poids de la solution, et
- de 0,3 à 3 mg/kg, de préférence de 0,5 à 7 mg/kg en poids de la solution, d'un stabilisant,
- optionnellement, de $1.10^{-4}$ % à $1.10^{-2}$ %, en poids de la solution, d'additif(s),
- du complément à 100 % d'eau.

**11.** Utilisation de la solution aqueuse telle que définie selon l'une quelconque des revendications précédentes pour la désinfection/stérilisation d'un produit, en particulier d'un emballage.

**12.** Utilisation selon la revendication précédente, dans laquelle la désinfection/stérilisation dudit produit est réalisée par pulvérisation ou vaporisation de ladite solution sur ledit produit.

**13.** Procédé de désinfection/stérilisation d'un produit, en particulier d'un emballage, **caractérisé en ce que** ledit produit est mis en contact avec la solution aqueuse selon l'une quelconque des revendications 1 à 10.

**14.** Procédé selon la revendication 13, dans lequel la mise en contact est réalisée par pulvérisation ou vaporisation de ladite solution sur ledit produit.

## Patentansprüche

**1.** Wässrige Wasserstoffperoxidlösung, umfassend:

- 20 bis 98 %, bezogen auf das Gewicht der Lösung, Wasserstoffperoxid und
- 0,5 bis 7 mg/kg, noch weiter bevorzugt 1,5 bis 5 mg/kg, bezogen auf das Gewicht der Lösung, eines Stabilisators, **dadurch gekennzeichnet, dass** der Stabilisator aus der Familie der Aminopolycarbonsäuren (APCA) oder Salzen davon der allgemeinen Formel:

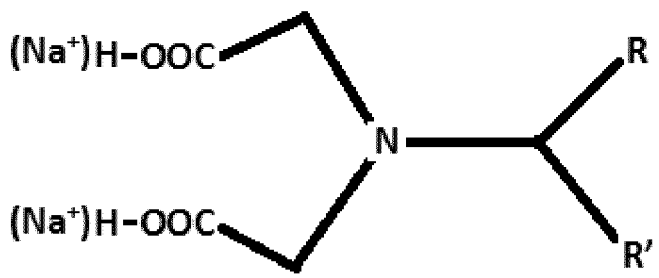

wobei R und R' aus den folgenden funktionellen Gruppen ausgewählt sind:

- C1- bis C3-Alkylgruppen, vorzugsweise einer Methylgruppe,
- C1-C3-Carbonsäuren,

ausgewählt ist.

2. Wässrige Lösung nach Anspruch 1, **dadurch gekennzeichnet, dass** sie 20 bis 75 %, vorzugsweise 20 % bis 65 %, vorzugsweise 20 bis 50 %, noch weiter bevorzugt 22 % bis 38 %, bezogen auf das Gewicht der Lösung, Wasserstoffperoxid umfasst.

3. Wässrige Lösung nach Anspruch 1 oder 2, **dadurch gekennzeichnet, dass** R oder R' aus einer Methylgruppe besteht und das andere R oder R' aus einer Methansäuregruppe oder einem Salz davon besteht, so dass der Stabilisator aus Methylglycindiessigsäure oder einem Salz davon besteht.

4. Wässrige Lösung nach Anspruch 1 oder 2, **dadurch gekennzeichnet, dass** R oder R' aus einer Ethansäuregruppe oder einem Salz davon besteht und das andere R oder R' aus einer Propansäuregruppe oder einem Salz davon besteht, so dass der Stabilisator aus Diessigsäureglutamat oder einem Salz davon besteht.

5. Wässrige Lösung nach einem der vorhergehenden Ansprüche, **dadurch gekennzeichnet, dass** sie eine Leitfähigkeit von 10 bis 100 $\mu$S/cm, vorzugsweise von 15 bis 50 $\mu$S/cm und noch weiter bevorzugt von 20 bis 40 $\mu$S/cm aufweist.

6. Wässrige Lösung nach einem der vorhergehenden Ansprüche, **dadurch gekennzeichnet, dass** sie eine maximale Acidität von 1 mmol/kg aufweist.

7. Wässrige Lösung nach einem der vorhergehenden Ansprüche, **dadurch gekennzeichnet, dass** sie einen Trockenrückstandsgehalt bei 110 °C von höchstens 7 mg/kg, vorzugsweise weniger als 6 mg/kg, vorzugsweise weniger als 5 mg/kg, vorzugsweise weniger als 4 mg/kg, vorzugsweise weniger als 3 mg/kg und noch weiter bevorzugt weniger als 2 mg/kg aufweist.

8. Wässrige Lösung nach einem der vorhergehenden Ansprüche, **dadurch gekennzeichnet, dass** der nach der CEFIC-$H_2O_2$-AM-7161-Methode gemessene Titerverlust der Lösung kleiner oder gleich 5 %, vorzugsweise kleiner oder gleich 3 % und bevorzugt kleiner oder gleich 2 %, bezogen auf die Anfangskonzentration an Wasserstoffperoxid, ist.

9. Wässrige Lösung nach einem der vorhergehenden Ansprüche, **dadurch gekennzeichnet, dass** der Stabilisator 0,1 bis 5 mg/kg, bezogen auf das Gewicht der Lösung, vorzugweise 1 bis 2,5 mg/kg, bezogen auf das Gewicht der Lösung, ausmacht.

10. Wässrige Lösung nach einem der vorhergehenden Ansprüche, umfassend:

    - 20 bis 50 % Wasserstoffperoxid, bezogen auf das Gewicht der Lösung, und
    - 0,3 bis 3 mg/kg, vorzugsweise 0,5 bis 7 mg/kg, bezogen auf das Gewicht der Lösung, eines Stabilisators,
    - gegebenenfalls $1 \times 10^{-4}$ % bis $1 \times 10^{-2}$ %, bezogen auf das Gewicht der Lösung, Additiv(e),
    - Rest auf 100 % Wasser.

11. Verwendung der wässrigen Lösung gemäß einem der vorhergehenden Ansprüche zur Desinfektion/Sterilisation eines Produkts, insbesondere einer Verpackung.

12. Verwendung nach dem vorhergehenden Anspruch, wobei die Desinfektion/Sterilisation des Produkts durch Aufsprühen oder Aufdampfen der Lösung auf das Produkt durchgeführt wird.

13. Verfahren zur Desinfektion/Sterilisation eines Produkts, insbesondere einer Verpackung, **dadurch gekennzeichnet, dass** das Produkt mit der wässrigen Lösung nach einem der Ansprüche 1 bis 10 in Kontakt gebracht wird.

14. Verfahren nach Anspruch 13, bei dem das Inkontaktbringen durch Aufsprühen oder Aufdampfen der Lösung auf das Produkt durchgeführt wird.

## Claims

1. Aqueous hydrogen peroxide solution comprising:

   - from 20 to 98%, by weight of the solution, of hydrogen peroxide, and
   - from 0.5 to 7 mg/kg and more preferentially from 1.5 to 5 mg/kg, by weight of the solution, of a stabilizer, **characterized in that** the stabilizer is chosen from the family of aminopolycarboxylic acids (APCA), or salts thereof, of general formula:

   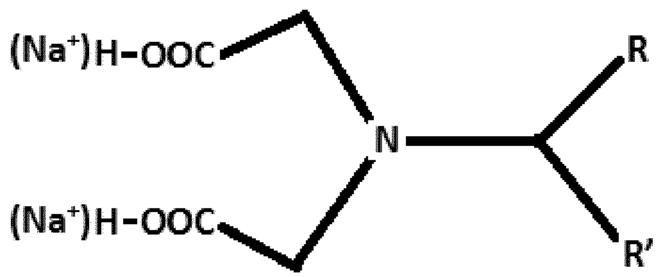

   in which R and R' are chosen from the following functional groups:

   - C1 to C3 alkyls, preferably a methyl group,
   - C1 to C3 carboxylic acids.

2. Aqueous solution according to Claim 1, **characterized in that** it comprises from 20 to 75%, preferably from 20% to 65%, preferably from 20 to 50% and more preferentially from 22% to 38%, by weight of the solution, of hydrogen peroxide.

3. Aqueous solution according to Claim 1 or 2, **characterized in that** R or R' consists of a methyl group and the other R or R' consists of a methanoic acid group or a salt thereof, such that the stabilizer consists of methylglycinediacetic acid or a salt thereof.

4. Aqueous solution according to Claim 1 or 2, **characterized in that** R or R' consists of an ethanoic acid group or a salt thereof, and the other R or R' consists of a propanoic acid group or a salt thereof, such that the stabilizer consists of diacetic acid glutamate or a salt thereof.

5. Aqueous solution according to any one of the preceding claims, **characterized in that** it has a conductivity of 10 to 100 $\mu$S/cm, preferentially from 15 to 50 $\mu$S/cm and even more preferably from 20 to 40 $\mu$S/cm.

6. Aqueous solution according to any one of the preceding claims, **characterized in that** it has a maximum acidity of 1 mmol/kg.

7. Aqueous solution according to any one of the preceding claims, **characterized in that** it has a content of dry residue at 110°C of not more than 7 mg/kg, preferably less than 6 mg/kg, preferably less than 5 mg/kg, preferably less than 4 mg/kg, preferably less than 3 mg/kg and more preferentially less than 2 mg/kg.

8. Aqueous solution according to any one of the preceding claims, **characterized in that** the loss of titer of said solution, measured according to the CEFIC-$H_2O_2$-AM-7161 method, is less than or equal to 5%, preferably less than or equal to 3% and preferably less than or equal to 2% relative to the initial concentration of hydrogen peroxide.

9. Aqueous solution according to any one of the preceding claims, **characterized in that** the stabilizer represents from 0.1 to 5 mg/kg, by weight of the solution, preferably from 1 to 2.5 mg/kg, by weight of the solution.

10. Aqueous solution according to any one of the preceding claims, comprising:

    - from 20% to 50% of hydrogen peroxide, by weight of the solution, and
    - from 0.3 to 3 mg/kg and preferably from 0.5 to 7 mg/kg, by weight of the solution, of a stabilizer,
    - optionally, from $1\times10^{-4}$% to $1\times10^{-2}$%, by weight of the solution, of additive(s),

- the remainder to 100% of water.

**11.** Use of the aqueous solution as defined in any one of the preceding claims for the disinfection/sterilization of a product, in particular of a packaging.

**12.** Use according to the preceding claim, in which the disinfection/sterilization of said product is performed by spraying or vaporizing said solution onto said product.

**13.** Process for disinfecting/sterilizing a product, in particular a packaging, **characterized in that** said product is placed in contact with the aqueous solution according to any one of Claims 1 to 10.

**14.** Process according to Claim 13, in which the placing in contact is performed by spraying or vaporizing said solution onto said product.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1926502 A
- WO 2015078830 A
- WO 2010004161 A
- EP 10177812 A
- WO 2010073976 A
- US 5759440 A